# EUROPEAN PATENT APPLICATION

(11) **EP 3 473 163 A1**
(43) Date of publication of application: **24.04.2019**
(21) Application number: 17197195.5
(22) Date of filing: 19.10.2017
(51) Int. Cl.: A61B 5/00, G01N 33/50

(54) **MODEL FOR EVALUATION OF DERMAL FILLERS**

(71) Applicant: Galderma Research & Development, 06410 Biot (FR)
(72) Inventor: OSMAN-PONCHET, Hanan, 06600 ANTIBES (FR)
(74) Representative: Axe PI

(57) **Abstract**

The invention relates to a model of skin sample. The model is *ex vivo* human skin and coupled with injection of dermal filler in the skin.

The invention also relates to use the model of skin of the invention to visualize effect of the dermal filler in the skin and to measure skin parameters and skin parameters changes before and after dermal filler injection and methods of use of the model of the invention.

## Description

The invention concerns a new model using *ex vivo* human skin samples to evaluate dermal fillers. More specifically the invention concerns a model of human skin coupled with injection of dermal filler. This new model is a cost-effective, rapid and simple tool suitable to monitor skin parameter changes induced by dermal filler injection as well as the visualization of dermal filler within skin samples.

In recent decades, injectable dermal fillers are becoming very useful for the correction of congenital or traumatic facial defects and in patients suffering from lipodystrophy following AIDS. Moreover, these substances are becoming very popular for the treatment of facial wrinkle(s).

There are numerous dermal fillers available based on different materials, and their properties differ both between and among classes. The model of *ex vivo* skin sample according to the invention can be used to visualize all types of existing filler. For examples, fillers can be chosen from collagen and derivates, hyaluronic acid, its salts and derivates, in a free form or crosslinked, alginates, synthetic polymers, elastin, biologic polymer and their mixtures.

Hyaluronic acid (HA) based fillers have varied life spans ranging from weeks to months depending upon their degree of reticulation. Highly reticulated HA fillers are more resistant to *in situ* degradation and show clinical efficacy for up to 1 year.

Current non-clinical assessment of dermal filler performance mainly involves *in vitro* analyses, which are poorly indicative, or *in vivo* implantation studies in animal models which are time consuming and are less and less used due to the regulatory context. In fact, the Cosmetics Directive establishes a prohibition to test finished cosmetic products and cosmetic ingredients on animals (testing ban), and a prohibition to market in the European Community finished cosmetic products and ingredients included in cosmetic products which were tested on animals (marketing ban). The testing ban on finished cosmetic products applies since 11 September 2004; the testing ban on ingredients or combination of ingredients applies since 11 March 2009. The marketing ban applies since 11 March 2009 for all human health effects with the exception of repeated-dose toxicity, reproductive toxicity and toxicokinetics. For these specific health effects the marketing ban will apply step by step as soon as alternative methods are validated and adopted in EU legislation with due regard to the OECD validation process, but with a maximum cut-off date of 10 years after entry into force of the Directive, *i.e*., 11 March 2013, irrespective of the availability of alternative non-animal tests.

In addition to prohibition, differences exist between animal skin and human skin which also motivate the man skilled in the art to find alternative way of testing. Although animal skin are used to test different compounds in pharmaceutical research, it has been found to be very different compared to human skin with regard to:
- Skin touch: animal skin is hairy, thus not suitable for cosmetic testing;
- Skin elasticity: minipig skin is less elastic compared to human skin, thus less suitable for dermal filler testing *in vitro*;
- Skin thickness: epidermis in mouse, rat and rabbit is too thin and leads to difficulties to perform injection. In minipig skin epidermis is thicker however, the very low elasticity does not allow easy injection.

For all the reasons mentioned above and for ethical considerations, there is a need to find alternative methods to test dermal filler in a method that mimics the real condition of use of the filler to do the best to characterize their behaviour and properties in the human skin *in vivo.*

A new model for investigation of dermal filler is described in the present invention.

Consequently, a first object of the solution proposed by the invention to remedy this problem is to provide a model of skin sample wherein the model is *ex vivo* human skin and coupled with injection of dermal filler in the skin.

Thus, a second object is to use the model of skin of the invention to visualize effect of the dermal filler in the skin.

A third object is to use the model of skin of the invention to measure skin parameters and skin parameters changes before and after dermal filler injection.

Other objects of the invention are methods of use of the model of skin of the invention respectively to visualize effect of the dermal filler in the skin; to measure skin parameters and skin parameters changes before and after dermal filler injection; to compare the diffusion and distribution of various dermal fillers in the skin; to measure the early inflammatory response due to dermal filler injection; and to measure the collagen synthesis due to dermal filler injection.

Finally, other objects of the invention are methods of using a model consisting of *ex vivo* skin sample coupled with dermal filler injection for respectively visualizing effect of the dermal filler in the skin, and measuring skin parameters changes after injection of a dermal filler in the skin.

The invention shall be better understood when reading the following non-restricted description, with reference to the accompanying drawings, wherein:
- Figures 1A-1B show how to inject dermal filler in the dermis of *ex vivo* human skin; Figures 1C-1E show different kind and volume of intradermal injection of dermal filler in *ex vivo* human skin;
- Figure 2A shows an *ex vivo* human skin sample after intradermal localized injection of dermal filler and Figures 2B-2C show resulting 3D digital photography views;
- Figures 3A-3B show slides of minipig skin respectively epidermal face and dermal face, after intradermal injection of dermal filler in *ex vivo* minipig skin;
- Figure 4 shows images by high frequency ultrasound of *ex vivo* human skin after injection of dermal filler;
- Figures 5A-5D show ultrasound images of *ex vivo* human skin samples after injection of dermal filler by comparing fresh and frozen skin samples;
- Figures 6A-6D show ultrasound images of *ex vivo* human skin samples after injection of dermal filler by comparing the volume of injection;
- Figures 7A-7D show ultrasound images of *ex vivo* human skin samples after injection of different dermal fillers;
- Figures 8A-8E show microphotographs of *ex vivo* human skin after injection of dermal filler by histopathology after skin section and special stain: Figures 8A-8B show comparison results of injection volumes and Figures 8C-8E show comparison results of different dermal fillers;
- Figure 9 shows visualization of dermal filler by RMI in *ex vivo* human skin;
- Figures 10A-10B show visualization of dermal filler by Explorer platform in *ex vivo* human skin: Figure 10A shows visualization with simultaneous measurement of viscoelasticity induced by the filler after intradermal injection and Figure 10B shows visualization with simultaneous measurement of viscoelasticity induced by the filler after intrahypodermal injection;
- Figure 11 shows an evaluation of skin visco-elastic properties and general skin firming after injection of dermal filler using the torsionnal ballistometer;
- Figure 12 shows an evaluation of skin thickness increase after injection of dermal filler in *ex vivo* human skin as measured by ultrasound; and
- Figure 13 shows comparison results of skin thickness induced by different dermal fillers.

According to the invention, the model claimed uses *ex vivo* human skin samples. Human skin samples are obtained from plastic surgery, from male or female, from various part of the human body. Preferably, human skin samples are obtained from abdominal, mammary or face skin.

Skin can be fresh, freezed or used freshly thawed. Preferably skin is fresh or freshly thawed.

Skin samples can be used with or without hypodermis. Depending of the parameters to be analysed after injection of the dermal filler in the skin, subcutaneaous fat can be removed carefully by dissection before using, to leave only dermis.

When prepared, skin samples are maintained in organoculture conditions up to three (3) weeks, preferably up to one (1) week, more preferably up to 120 hours.

According to the invention, the model of human skin is to be used to characterize injectable dermal filler. Injection of dermal filler in *ex vivo* human skin may be performed intradermally or intrahypodermally or both.

According to the invention, intradermally means within the dermis, intrahypodermally means within the subcatenous fat, when not removed. Both site of injection can be used.

Injection is performed using injection needle classically used in dermal filler injection. Preferably, injection is performed using a 30G1/2 needle.

As performed *in vivo* on human, injection of dermal filler in the *ex vivo* human skin sample may be a localized one or a linear retro-tracing one.

Injected volumes of dermal filler are comprised between 10 and 500 µL, preferably between 50 and 200 µL, more preferably equal to 50, 100 or 200 µL.

Intradermal injection of dermal filler in the skin sample according to the invention is done with dermal side down or dermal side up.

In the present invention, the *ex vivo* human skin sample necessary for injection of dermal filler represents a surface between 3 and 10 cm², preferably at minimum equal to 4 cm² (2 x 2 cm).

When injected with the dermal filler, the human skin according to the invention is used to characterize various parameters related to fillers and/or skin.
- Visualization of dermal filler diffusion within the skin (in the dermis and in the hypodermis);
- Measurement of skin change (thickness increase, area, volume, firming, elasticity) due to dermal filler;
- Measurement of dermal filler diffusion within the skin;
- Comparison of diffusion and distribution pattern of different dermal filler;
- Measurement of early inflammatory response due to dermal filler;
- Measurement of collagen synthesis change due to dermal filler.

Various techniques can be used in combination with the model according to the invention to visualize and/or measure these parameters before and after dermal filler injection.

The techniques usable to measure and visualize these parameters are listed below.

In one preferred embodiment of the invention, this measurement can be done with high frequency ultrasound or dermo-echography. Preferably, a 20 MHz probe is used. Dermo-echography coupled to the human skin sample according to the invention lead to a non-invasive method that allows indirect visualization of the dermal filler in the skin and allows measurement of the skin parameters before and after dermal filler injection. More preferably, human skin parameters which can be measured are skin thickness, skin area and/or volume.

In another embodiment, the human skin sample according to the invention can be coupled with histopathology. Preferably, histopathology is performed with image analysis, allowing direct visualization of dermal filler in skin sections, measurement of skin thickness changes, and visualization of the distribution of dermal filler within the dermal matrix.

Various other techniques can be used in combination with the model according to the invention to visualize volume changes, skin thickness changes, diffusion of the filler with time.

Optical Coherence Tomography (OCT) is a non-invasive method allowing visualization of dermal filler in the skin and measurement of skin thickness changes due to dermal filler.

3D digital photography is a non-invasive method allowing measurement of skin volume change due to dermal filler.

Resonnance Magnetic Imaging is a non-invasive method allowing direct visualization of dermal filler in the skin.

AixPlorer platform is a non-invasive method allowing indirect visualization of dermal filler in the dermis and also in the hypodermis and measurement of skin thickness changes due to dermal filler as well as measurement of skin viscoelasticity change due to dermal filler.

Ballistometer is a non-invasive method allowing measurement of skin firming and elasticity change due to dermal filler.

In one embodiment of the invention, the *ex vivo* human skin sample is used to measure the kinetics of diffusion of dermal filler in the skin. This evaluation of dermal filler in *ex vivo* human skin may be performed just after filler injection (T0) or several hours after filler injection up to three (3) weeks, preferably up to one (1) week, preferably up to 120 hours. In those cases, skin samples should be maintained in skin culture medium in cell culture incubator set at 37°C, 5% CO₂ and humidified atmosphere.

The following examples give some non-exhaustive examples of use of *ex vivo* human skin according to the invention.

### Example 1: Intradermal injection of dermal filler in ex vivo human skin

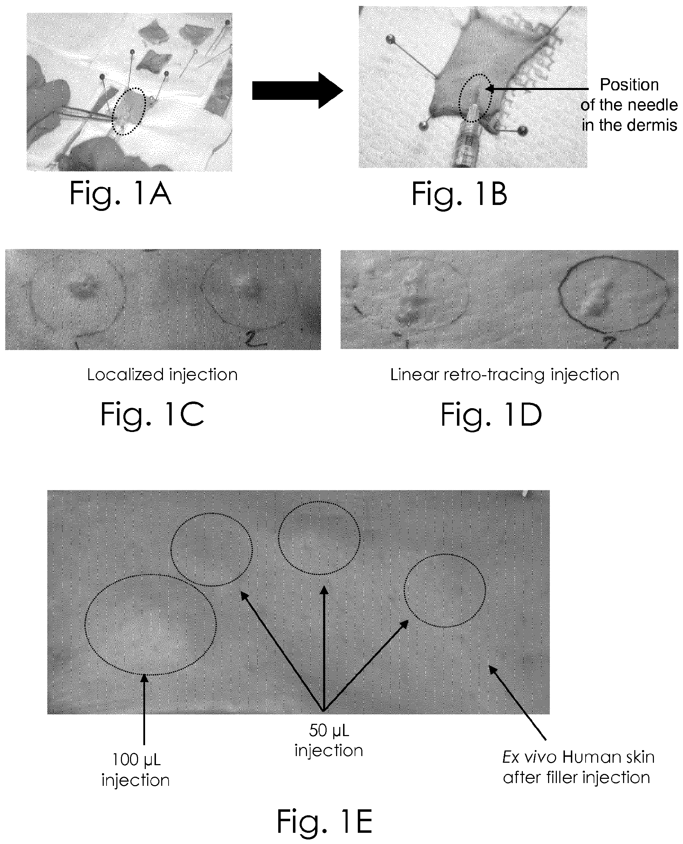

Figures 1A-1E show different kind and volume of intradermal injection of dermal filler in *ex vivo* human skin.

For example, the position of the needle during intradermal injection of a dermal filler in an *ex vivo* human skin sample is illustrated by Figures 1A-1B.

Figures 1C and 1D show more particularly the different aspects between respectively a localized injection and a linear retro-tracing injection, and Figure 1E of 50 µL and 100 µL of the dermal filler in an *ex vivo* human skin sample.

### Example 2: 3D digital photography

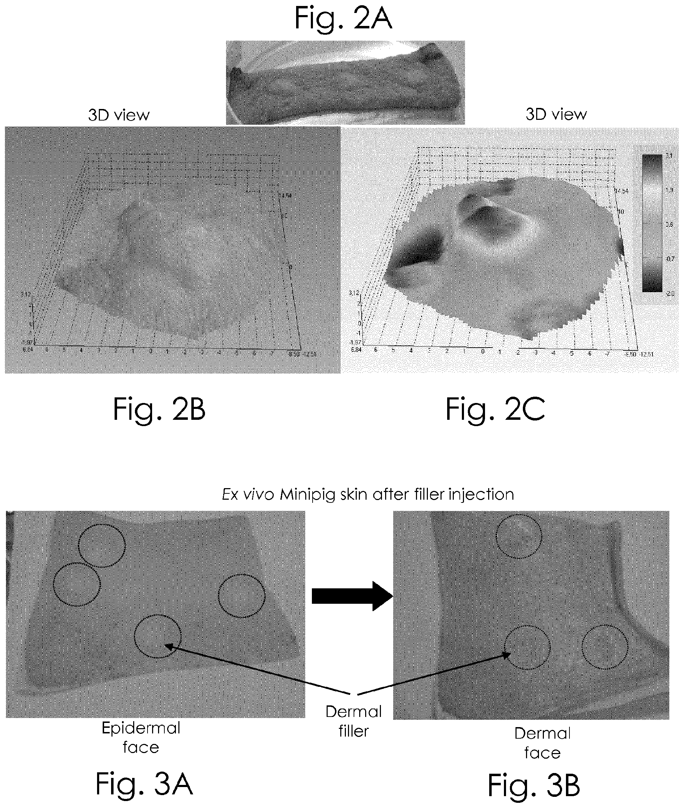

Figure 2A shows an *ex vivo* human skin sample after intradermal localized injection of dermal filler and Figures 2B-2C show resulting 3D digital photography views.

As illustrated, intradermal localized injection of dermal filler in *ex vivo* human skin samples induces an increase in skin volume and forms a hard ball into the skin as observed in 3D view.

### Example 3: Intradermal injection of dermal filler in ex vivo minipig skin

Figures 3A-3B show slides of minipig skin respectively epidermal face and dermal face, after intradermal injection of dermal filler in *ex vivo* minipig skin. The following observations can be made:
- Minipig skin is very less elastic than human skin.
- When injected intradermally, leakage of dermal filler is observed on the dermal face, through hair follicle base.
- *Ex vivo* minipig skin is not a suitable model to evaluate dermal filler diffusion.

### Example 4: Visualization of dermal filler by high frequency ultrasound

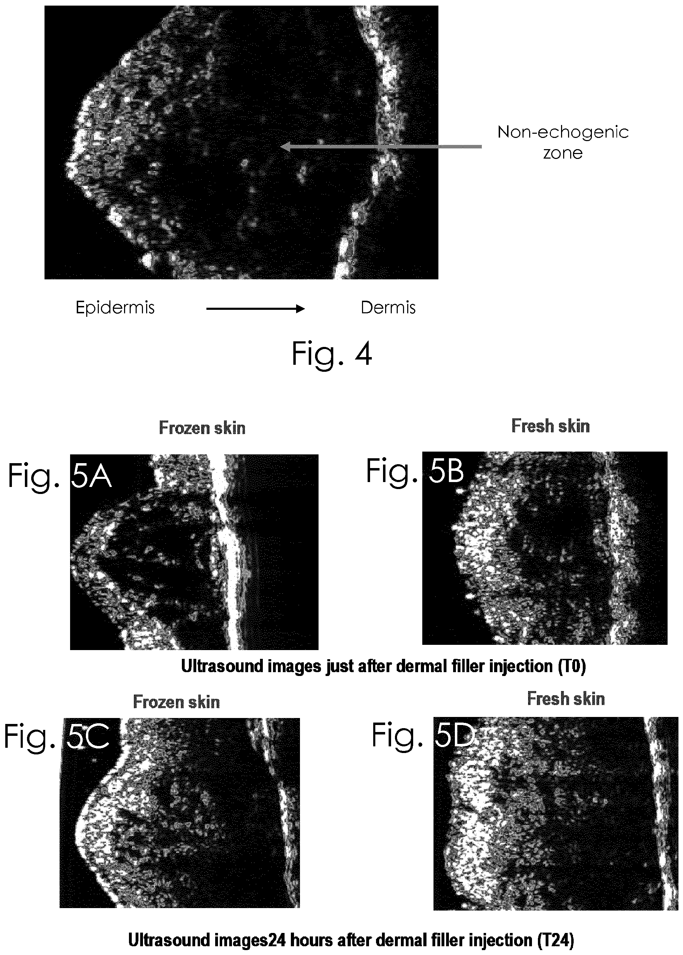

Figure 4 shows image by high frequency ultrasound of *ex vivo* human skin after injection of dermal filler;

Ultrasound image of the *ex vivo* human skin sample after injection of dermal filler shows three layer structures:
- Uppermost layer: echogenic
- Intermediate layer: poorly echogenic
- Dermis-hypodermis interface: echogenic

After injection of dermal filler, the dermis is largely thickened and appears less echogenic.

### Example 5: Ultrasound images of ex vivo human skin sample after injection of dermal filler. Comparison between fresh and frozen skin samples

As shown by Figures 5A-5D, no difference is observed between fresh and freshly thawed skin just after dermal filler injection (T0) or 24 hours after (T24).

### Example 6: Ultrasound images of ex vivo human skin sample after injection of dermal filler. Comparison of the volume of injection between 100 µL and 200 µL just after dermal filler injection (T0) or 24 hours after (T24)

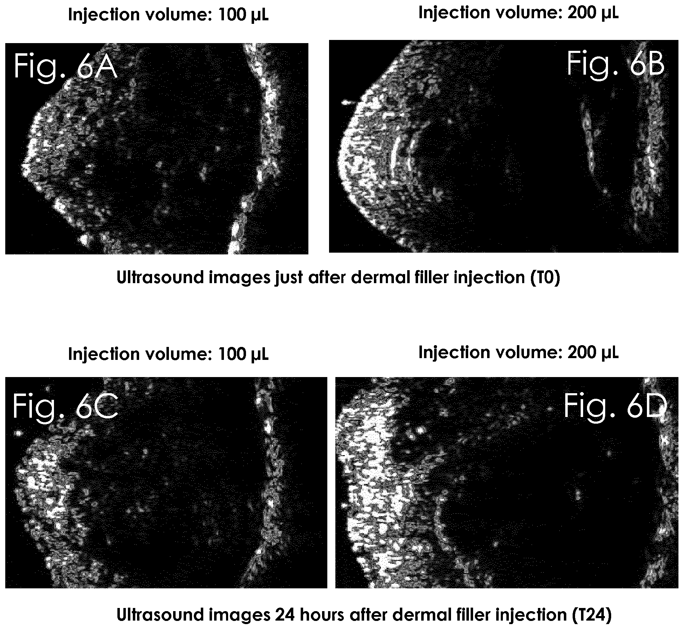

Ultrasound images after dermal filler injection of Figures 6A-6D show that:
1. The dermis is largely thickened.
2. The dermis appears less echogenic.
3. In some cases, totally non-echogenic zones are observed within the dermis probably corresponding to hyaluronic acid that is non-echogenic.

### Example 7: Ultrasound images of ex vivo human skin sample after injection of different dermal fillers just after dermal filler injection (T0)

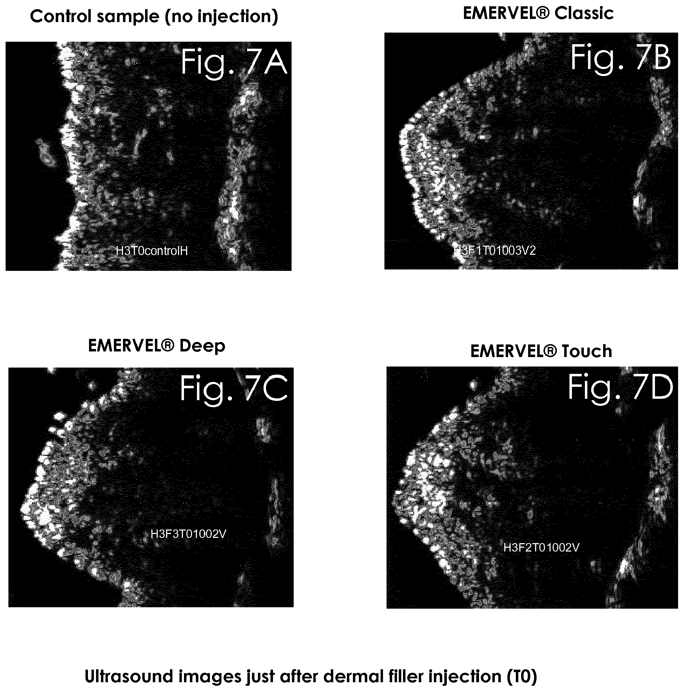

Ultrasound images after injection of EMERVEL® dermal fillers of Figures 7B-7D (by comparison to Figure 7A (control sample) show that:
1. The dermis is largely thickened.
2. The dermis appears less echogenic.
3. With the three dermal fillers of EMERVEL® range, totally non-echogenic zones are observed within the dermis probably corresponding to the dermis area occupied by hyaluronic acid (non-echogenic material).

### Example 8: Visualization of dermal filler by Histopathology after skin section and special stain

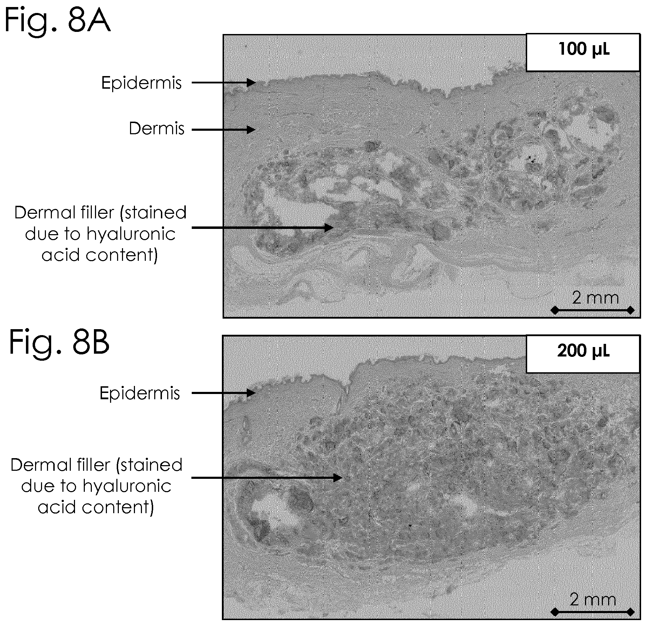

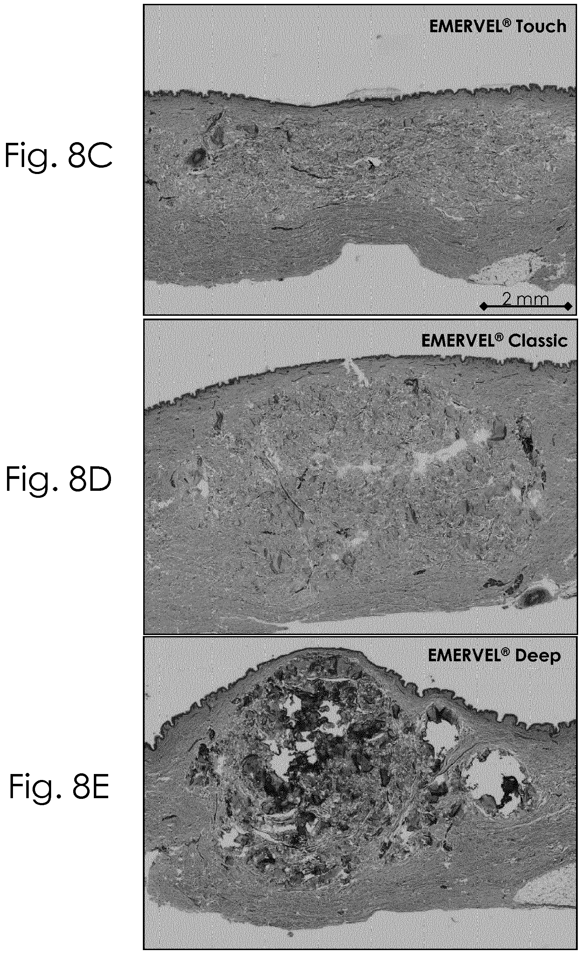

Figures 8A-8E show microphotographs of *ex vivo* human skin after injection of dermal filler by histopathology after skin section and special stain.

### 8a) Comparison of injection volumes (100 and 200 µL) at T24h

Figures 8A-8B show comparison results of injection volumes. As illustrated, dermal filler is clearly visible as it is stained [in blue - not applicable here due to black and white colors which have to be used] due to hyaluronic acid content.

At microscopic examination, the site of injection is well visible at low magnification in the dermis.

Measurement of the area (mm²) occupied by the dermal filler is performed by image analysis after color deconvolution and use of a binary mask in MATLAB®. A statistically significant discrimination is possible between 100 and 200 µL injection volumes (p < 0.001).

### 8b) Comparison of different dermal fillers

Figures 8C-8E show comparison results of different dermal fillers. As illustrated, respective dermal fillers are stained [in blue - not applicable here due to black and white colors which have to be used] due to hyaluronic acid content.

### Example 9: Visualization of dermal filler by RMI in ex vivo human skin

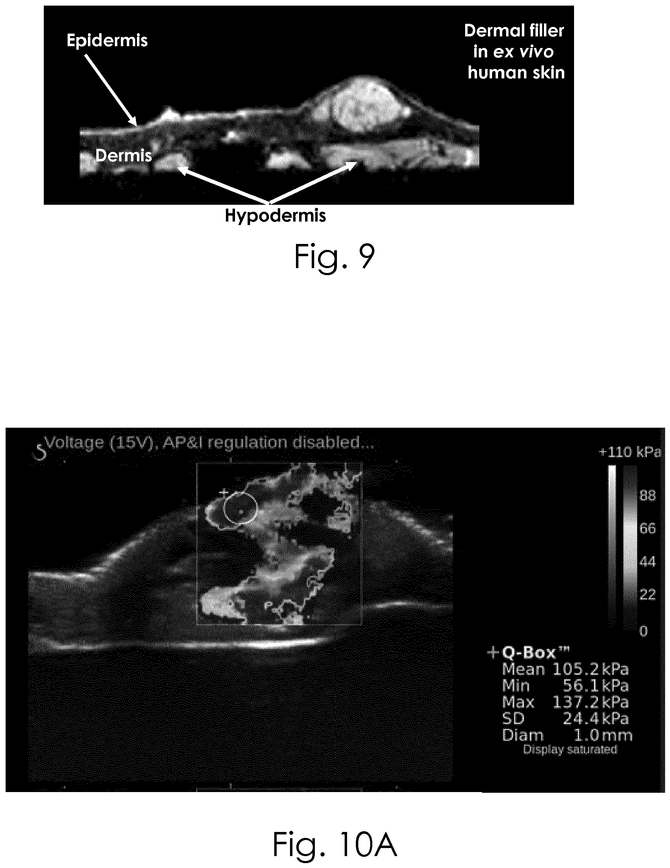

Figure 9 shows visualization of dermal filler by RMI in *ex vivo* human skin.

### Example 10: Visualization of dermal filler by Explorer platform

Figures 10A-10B show visualization of dermal filler by Explorer platform in *ex vivo* human skin.

10a) Figure 10A shows visualization of dermal filler in *ex vivo* human skin with simultaneous measurement of viscoelasticity induced by the filler after intradermal injection.

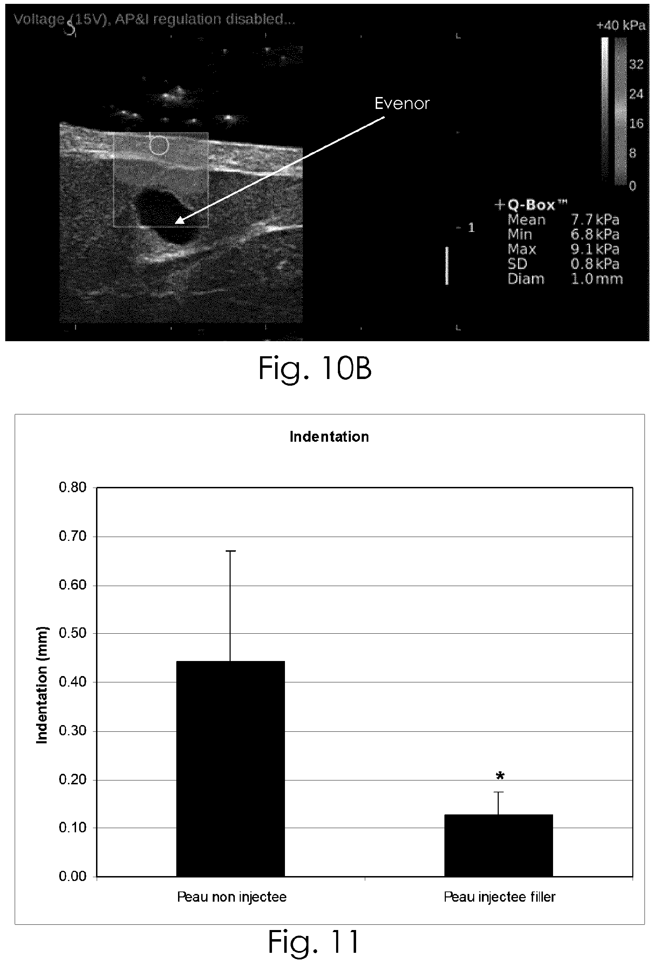

10b) Figure 10B shows visualization of dermal filler in ex vivo human skin with simultaneous measurement of viscoelasticity induced by the filler after intrahypodermal injection.

### Example 11: Evaluation of skin visco-elastic properties and general skin firming after injection of dermal filler using the torsionnal ballistometer

Figure 11 shows an evaluation of skin visco-elastic properties and general skin firming after injection of dermal filler using the torsionnal ballistometer;

Indentation is a measure of skin firming. A decrease of indentation value indicates an increase in skin firming.

As illustrated by Figure 11, injection of dermal filler induces a statistically significant decrease in indentation, indicating a significant increase in skin firming as measured by ballistometer.

### Example 12: Evaluation of skin thickness increase after injection of dermal filler in ex vivo human skin as measured by ultrasound

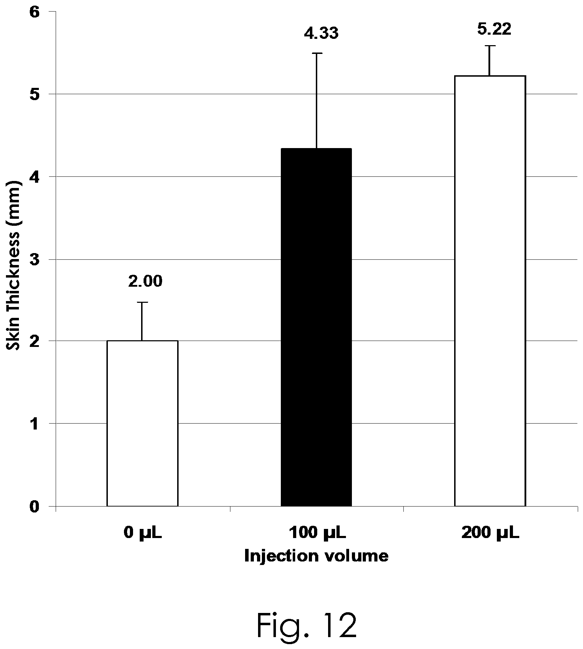

As illustrated by Figure 12:
- Dermal filler injection in *ex vivo* human skin induces a marked skin thickness increase that can be estimated by image analysis (Dermascan Software).
- Relative to the concurrent control (0 µL), skin thicknesses increase by a 2.2-fold factor after 100 µL injection and by a 2.6-fold factor after 200 µL injection at T0 (n = 3 donors).
- There is a low inter-individual variability in skin thickness, with CV ranging between 7% and 27%.

### Example 13: Comparison of skin thickness induced by different dermal filler

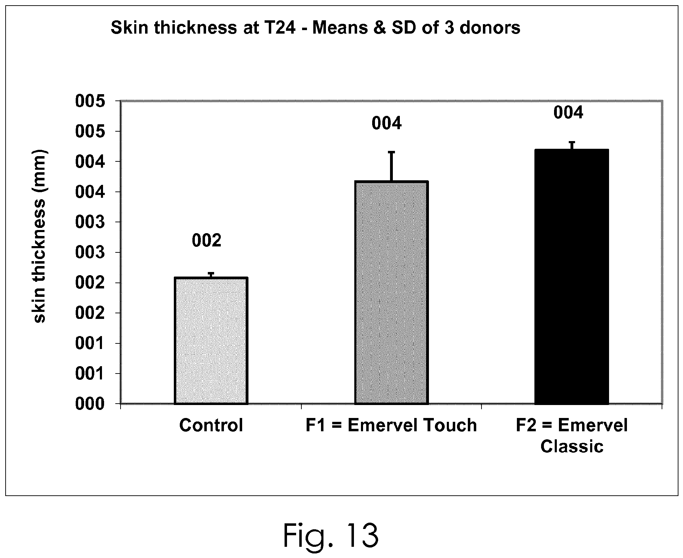

As illustrated by Figure 13, a significative difference between EMERVEL® Touch and EMERVEL® Classic (Student Test, p<0.05) is observed: a lower increase of skin thickness is observed with EMERVEL® Touch (the less reticulated filler) compared to EMERVEL® Classic.

## Claims

1. A model of skin sample wherein the model is *ex vivo* human skin and coupled with injection of dermal filler in the skin.

2. The model according to claim 1, wherein the human skin is fresh, freezed, or freshly thawed.

3. The model according to claim 1 or 2, wherein the human skin comprises the dermis.

4. The model according to any of claims 1 to 3, wherein the human skin comprises the dermis and the hypodermis.

5. Use of the model of any of claims 1 to 4 to visualize effect of the dermal filler in the skin.

6. Use of the model of any of claims 1 to 4 to measure skin parameters and skin parameters changes before and after dermal filler injection.

7. The use according to claim 6, wherein the skin parameters are thickness, area, volume, firming, elasticity.

8. A method of use of the model according to any of claims 1 to 4 to visualize effect of the dermal filler in the skin.

9. A method of use of the model according to any of claims 1 to 4 to measure skin parameters and skin parameters changes before and after dermal filler injection.

10. A method of use of the model according to any of claims 1 to 4 to compare the diffusion and distribution of various dermal fillers in the skin.

11. A method of use of the model according to any of claims 1 to 4 to measure the early inflammatory response due to dermal filler injection.

12. A method of use of the model according to any of claims 1 to 4 to measure the collagen synthesis due to dermal filler injection.

13. A method of visualizing effect of the dermal filler in the skin, said method using a model consisting of *ex vivo* skin sample coupled with dermal filler injection according to any of claims 1 to 4.

14. A method of measuring skin parameters changes after injection of a dermal filler in the skin, said method using a model consisting of *ex vivo* skin sample coupled with dermal filler injection according to any of claims 1 to 4.
